(19)

Europäisches
Patentamt
European
Patent Office
Office européen
des brevets

(11)   **EP 2 442 840 B1**

(12)   **EUROPEAN PATENT SPECIFICATION**

(45) Date of publication and mention
of the grant of the patent:
**30.10.2013 Bulletin 2013/44**

(21) Application number: **10727273.4**

(22) Date of filing: **08.06.2010**

(51) Int Cl.:
**A61L 29/08** (2006.01)          **A61L 29/14** (2006.01)
**A61L 29/16** (2006.01)

(86) International application number:
**PCT/US2010/037799**

(87) International publication number:
**WO 2010/147805 (23.12.2010 Gazette 2010/51)**

(54) **DRUG COATED BALLOON CATHETER AND PHARMACOKINETIC PROFILE**

WIRKSTOFFBESCHICHTETER BALLONKATHETER UND PHARMAKOKINETISCHES PROFIL

CATHÉTER À BALLONNET REVÊTU DE MÉDICAMENT ET PROFIL PHARMACOCINÉTIQUE

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **17.06.2009 US 486652**

(43) Date of publication of application:
**25.04.2012 Bulletin 2012/17**

(73) Proprietor: **Abbott Cardiovascular Systems Inc.
Santa Clara, CA 95054-2807 (US)**

(72) Inventors:
• **STANKUS, John, J.**
**Campbell, CA 95008 (US)**
• **TROLLSAS, Mikael**
**San Jose, CA 95124 (US)**
• **HOSSAINY, Syed**
**San Jose, CA 94555 (US)**
• **ZHANG, Liangxuan**
**Palo Alto, CA 94306 (US)**
• **BERGER, Ed**
**San Jose, CA 95112 (US)**
• **PACETTI, Stephen, D.**
**San Jose, CA 95130 (US)**
• **TONER, John, L.**
**Libertyville, IL 60048 (US)**

(74) Representative: **Jackson, Martin Peter
J A Kemp
14 South Square
Gray's Inn
London WC1R 5JJ (GB)**

(56) References cited:
**WO-A1-2007/065722          WO-A1-2009/051614
WO-A2-2010/030995**

• **BURKE S E ET AL: "Zotarolimus (ABT-578)
eluting stents", ADVANCED DRUG DELIVERY
REVIEWS, ELSEVIER BV, AMSTERDAM, NL, vol.
58, no. 3, 3 June 2006 (2006-06-03), pages 437-446,
XP024892174, ISSN: 0169-409X, DOI: DOI:
10.1016/J.ADDR.2006.01.021 [retrieved on
2006-06-03]**
• **B. SCHELLER ET AL.: "Paclitaxel Balloon
Coating, a Novel Method for Prevention and
Therapy of Restenosis", CIRCULATION, vol. 110,
9 August 2004 (2004-08-09), pages 810-814,
XP002614503,**
• **J. C. FANGGIDAY ET AL.: "Safety and Efficacy of
Drug-Eluting Balloons in Percutaneous
Treatment of Bifurcation Lesions: The DEBIUT
(Drug-Eluting Baloon in BIfurcaton UTrecht)
Registry", CATHETERIZATION AND
CARDIOVASCULAR INTERVENTIONS, vol. 71, 1
January 2008 (2008-01-01), pages 629-635,
XP002614504,**
• **TAISUKE MORI ET AL: "Retention of paclitaxel in
cancer cells for 1 week in vivo and in vitro",
CANCER CHEMOTHERAPY AND
PHARMACOLOGY, SPRINGER, BERLIN, DE, vol.
58, no. 5, 14 March 2006 (2006-03-14) , pages
665-672, XP019423323, ISSN: 1432-0843, DOI:
DOI:10.1007/S00280-006-0209-6**

**EP 2 442 840 B1**

- B. SCHELLER ET AL.: "Treatment of Coronary In-Stent Restenosis with a Paclitaxel-Coated Balloon Catheter", THE NEW ENGLAND JOURNLA OF MEDICINE, vol. 355, no. 20, 16 November 2006 (2006-11-16), pages 2113-2123, XP002614505,

- MAIER L S ET AL: "Hotline update of clinical trials and registries presented at the German Cardiac Society meeting 2008; (PEPCAD, LokalTax, INH, German ablation registry, German device registry, DES.DE registry, DHR, Reality, SWEETHEART registry, ADMA, GERSHWIN)", CLINICAL RESEARCH IN CARDIOLOGY, STEINKOPFF-VERLAG, DA, vol. 97, no. 6, 25 April 2008 (2008-04-25) , pages 356-363, XP019628416, ISSN: 1861-0692

**Description**

## FIELD OF THE INVENTION

The present invention is related to the delivery of drugs from a coated angioplasty balloon and the pharmacokinetic profile of the released drug from the tissue.

## BACKGROUND OF THE INVENTION

Atherosclerosis is a syndrome affecting arterial blood vessels. It is a chronic inflammatory response in the walls of arteries, in large part due to the accumulation of blood cells and promoted by low density lipoproteins and the formation of plaque on the arterial wall. Atherosclerosis is commonly referred to as hardening of the arteries. Angioplasty is a vascular interventional technique involving mechanically widening an obstructed blood vessel, typically caused by atherosclerosis.

During angioplasty, a catheter having a tightly folded balloon is inserted into the vasculature of the patient and is passed to the narrowed location of the blood vessel at which point the balloon is inflated to a fixed size using fluid pressures. Percutaneous coronary intervention (PCI), commonly known as coronary angioplasty, is a therapeutic procedure to treat the stenotic coronary arteries of the heart, often found in coronary heart disease.

Peripheral angioplasty, commonly known as percutaneous transluminal angioplasty (PTA), refers to the use of mechanical widening of blood vessels other than the coronary arteries. PTA is most commonly used to treat narrowing of the leg arteries, especially, the iliac, external iliac, superficial femoral and popliteal arteries. PTA can also treat narrowing of veins, and other blood vessels.

It was found that following angioplasty, although a blood vessel would be successfully widened, sometimes the treated wall of the blood vessel becomes weakened after balloon inflation or dilatation, causing the blood vessel to collapse after the balloon is deflated or later. Interventional cardiologists addressed this problem by stenting the blood vessel to prevent collapse. A stent is a device, typically a metal tube or scaffold, that was inserted into the blood vessel following angioplasty, in order to hold the blood vessel open.

While the advent of stents eliminated many of the complications of abrupt blood vessel collapse after angioplasty procedures, it was found that within about six months of stenting a re-narrowing of the blood vessel often persisted, a condition known as restenosis. Restenosis was discovered to be a "controlled injury" of the angioplasty procedure and was characterized by a growth of smooth muscle cells-analogous to a scar forming over an injury. It was thought that drug eluting stents were the answer to the reoccurrence of narrowing of blood vessels after stent implantation. A drug eluting stent is a metal stent that has been coated with a drug that is known to interfere with the process of re-narrowing of the blood vessel (restenosis).

One drawback of drug eluting stents is a condition known as late stent thrombosis, an event in which blood clots inside the stent. Thrombosis is fatal in over one-third of cases. Drug eluting balloons are believed to be a viable alternative to drug eluting stents in the treatment of atherosclerosis. In a study which evaluated restenosis and the rate of major adverse cardiac events such as heart attack, bypass, repeat stenosis, or death in patients treated with drug eluting balloons and drug eluting stents, the patients treated with drug eluting balloons experienced only 3.7 percent restenosis and 4.8% MACE as compared to patients treated with drug eluting stents, in which restenosis was 20.8 percent and 22.0 percent MACE rate. (See, PEPCAD II study, Rotenburg, Germany).

Although drug eluting balloons are a viable alternative and in some cases appear to have greater efficacy than drug eluting stents as suggested by the PEPCAD II study, drug eluting balloons present challenges due to the very short period of contact between the drug coated balloon surface and the blood vessel wall. In particular, a non-perfusion balloon can only be inflated for less than one minute,  and is often inflated for only thirty seconds which would otherwise starve distal regions of oxygenated blood. Therefore, an efficacious, therapeutic amount of drug must be transferred to the vessel wall within a thirty second to one minute time period. Thus, there are challenges specific to drug delivery via a drug coated balloon because of the necessity of a short inflation time, and therefore time for drug or coating transfer--a challenge not presented by a drug eluting stent, which remains in the patient's vasculature once implanted.

Other considerations are the current theories about the mechanism by which a drug coated balloon transfers drug to the vessel wall. One theory, for example, is that upon balloon expansion, the drug composition mechanically fractures or dissolves from the coating, diffuses to the vessel wall and then permeates into the vessel wall. A second theory is that upon balloon expansion the balloon coating is transferred to the vessel wall, and then drug permeates into the vessel wall from the coating adhered to the vessel wall. Another theory is that the balloon expansion creates tears and microfissures in the vessel wall and portions of the coating insert into the tears and microfissures. The drug then permeates into the vessel wall from the coating within the tears and fissures. Yet another theory is that upon balloon expansion, a layer of dissolved drug and coating excipients is formed at a high concentration on the vessel wall as a boundary layer. The drug diffuses and permeates from this boundary layer into the vessel wall. In most of these theories, the drug transfers from the balloon to the circulation or the vascular wall tissue upon fracture of the coating due to inflation of the balloon and occurs within one minute, and preferably within 30 seconds. Once the diffused drug is within the vessel tissue, the initial high concentration of drug serves as a reservoir which diffuses into the other surrounding vessel tissue, thereby exhibiting a characteristic pharmacokinetic (PK) release profile. Therefore, a need exists for a drug coated balloon having efficient drug transfer to a vessel wall.

Various embodiments of DC balloons have been proposed, including balloons with a therapeutic agent disposed directly on the balloon surface and balloons having various protective sheaths. However, not all embodiments result in an efficacious response in reducing restenosis after balloon and bare metal stent trauma. WO 2009/051614 discloses drug-releasing coatings for medical devices.

Therefore, a need exists for a drug eluting balloon and more particularly, a balloon coated with a cytostatic therapeutic agent, that provides for an effective pharmacokinetic (PK) profile of drug tissue concentration over time after delivery from this coated balloon.

## SUMMARY OF INVENTION

The purpose and advantages of the disclosed subject matter will be set forth in and apparent from the description that follows, as well as will be learned by practice of the disclosed subject matter. Additional advantages of the invention will be realized and attained by the methods and systems particularly pointed out in the written description and claims hereof, as well as from the appended drawings.

The present invention provides a drug delivery balloon comprising:

a balloon having a surface; and
a coating disposed on at least a portion of the surface, wherein the coating includes a cytostatic therapeutic agent, an excipient, and a plasticizer, wherein at least 30% of the coating transfers from the balloon surface within two minutes after inflation of the balloon, and further wherein the coating produces a peak tissue concentration of the cytostatic therapeutic agent in the range of 10-1000 ng/mg of tissue approximately 24 hours after inflation of the balloon, and further wherein the cytostatic drug is everolimus, and the everolimus has a concentration on the balloon between 88 to 1500 ug/cm2 or between 100 to 600 ug/cm2, or wherein the cytostatic drug is zotarolimus, and the zotarolimus has a concentration on the balloon between 15 to 1500 ug/cm2.

The present invention also provides a drug delivery balloon comprising:

a perfusion balloon having a surface; and
a coating disposed on at least a portion of the surface, wherein the coating includes having an cytostatic therapeutic agent, an excipient, and a plasticizer, and further wherein at least 30% of the coating transfers from the balloon surface within ten minutes after inflation of the balloon, or within 5 minutes of inflation, or within 2 minutes of inflation or less, and further wherein the cytostatic drug is everolimus, and the everolimus has a concentration on the balloon between 88 to 1500 ug/cm2 or between 100 to 600 ug/cm2, or wherein the cytostatic drug is zotarolimus, and the zotarolimus has a concentration on the balloon between 15 to 1500 ug/cm2.

In accordance with another embodiment, at least 50% of the coating transfers from the balloon surface within two minutes after inflation of the balloon. In accordance with yet another embodiment, at least 90% of the coating transfers from the balloon surface within two minutes after inflation of the balloon.

In accordance with the present subject matter, a sufficient drug concentration is deposited at the targeted tissue site of interest such that the resulting pharmacokinetic (PK) profile, or decline in tissue concentration with time, can provide the local drug concentration necessary to inhibit restenosis.

In accordance with a preferred embodiment of the invention, the excipient is biocompatible. For example, some non-limiting examples of suitable excipients include polyvinylpyrrolidone (PVP), polysorbates such as Tween 80® or Tween 20®, polyethylene glycol or any combination thereof. In one embodiment, the PVP is preferably not substantially cross-linked and preferably is not a hydrogel. The plasticizer is preferably biocompatible. Some nonlimiting suitable plasticizers glycerol, ethanol, polyethylene glycol, propylene glycol, benzyl alcohol, N-methylpyrrolidone, Cremophor EL, dimethylsulfoxide, water, sucrose, sorbitol, or a blend thereof.

A balloon catheter is disclosed for delivering a therapeutic agent to the vasculature of a patient and also other target tissues. The tissue can be a blood tissue, a blood vessel (such as a peripheral or coronary artery), or a blood vessel within an organ or a muscle.

In accordance with the invention, the coating of the drug delivery balloon is designed to produce a pK profile in the vasculature or target tissue that can provide controlled release of the cytostatic drug. Preferably, the pK profile provides for a local therapeutic agent concentration necessary to inhibit restenosis. In accordance with one embodiment of the invention, the coating achieves detectable amounts of the cytostatic drug in a tissue over a period of at least one week post delivery of the drug.

In accordance with a preferred embodiment, the coating of the drug delivery balloon produces a pK profile with a zotarolimus tissue concentrations half life in the range of 24 to 96 hours. In accordance with yet another embodiment, the coating of the drug delivery balloon produces a pK profile with an everolimus tissue concentration half life in the range of 18 to 48 hours. Preferably, and in accordance with the invention, the desired pK profile produces an acute tissue concentration in the range of 10-1000 ng/mg.

In accordance with one aspect, the coating of the drug delivery balloon includes everolimus, and the everolimus has a concentration between 88 to 1500 $\mu$g/cm$^2$, preferably 500 to 1500 $\mu$g/cm$^2$.

In accordance with another aspect, the coating of the drug delivery balloon includes zotarolimus and the zotarolimus has a concentration between 15 to 1500 $\mu$g/cm$^2$, preferably 15 to 600 ug/cm$^2$.

In accordance with one embodiment, the cytostatic concentration in the blood system increases as a function of time with $T_{max}$ ranging from 1 to 3 hours and $C_{max}$ ranging from 2 to 40 ng/mL or 0.02 to 0.05 ng/mL/ug as a function of coating formulation. In this regard, the zotarolimus concentration in the blood system does not exceed a $C_{max}$ 111 ng/ml 2 hours post balloon inflation when normalized to total dosage. In another embodiment, the cytostatic concentration normalized to a total dosage in a subject's blood does not exceed 0.1 ng/ml/ug 5 hours post inflation.

In accordance with a further embodiment, the drug delivery balloon is a perfusion balloon and includes a coating disposed on at least a portion of the surface of the perfusion balloon, wherein the coating includes having an cytostatic therapeutic agent, an excipient, and a plasticizer, and further wherein at least 30% of the coating transfers from the balloon surface within ten minutes after inflation of the balloon. In another embodiment, the drug delivery balloon can be mounted on a catheter with perfusion ports.

It is to be understood that both the foregoing description is exemplary and is intended to provide further explanation of the invention claimed to a person of ordinary skill in the art. The accompanying drawings are included to illustrate various embodiments of the invention to provide a further understanding of the invention. The exemplified embodiments of the invention are not intended to limit the scope of the claims.

## BRIEF DESCRIPTION OF DRAWINGS

The disclosed subject matter will now be described in conjunction with the accompanying drawings in which:

FIG. 1A is a planar view of a balloon catheter in accordance with the invention; and FIG. 1B is a cross-sectional view taken along lines A-A in FIG. 1A in accordance with one embodiment of the invention.

FIGS. 2A and 2B are graphs illustrating everolimus tissue concentrations as a function of tissue mass in Fig. 2a and arterial lumen surface area in Fig. 2b at 24 hours and 72 hours after delivery from everolimus, coated balloon in accordance with one embodiment of the present invention;

FIGS. 3A and 3B are graphs illustrating everolimus tissue concentrations in proximal and distal arterial tissues to the treated region after 24 (Figs 3a) and 72 (Figs 3a) hours post delivery in a porcine pharmacokinetic model using an embodiment of the present invention; and

FIG. 4 is a graph illustrating the normalized tissue uptake in the porcine pharmacokinetic model in accordance with an embodiment of the present invention;

FIG. 5. is a graph illustrating zotarolimus tissue concentrations (ng drug/mg tissue) as a function of time post zotarolimus-coated balloon delivery in a porcine model in accordance with one embodiment of the present invention;

FIG. 6 is a graph illustrating zotarolimus tissue concentrations as a function of time post zotarolimus:excipient coating balloon delivery in a porcine model in accordance with another embodiment of the present invention;

FIG. 7 is a graph illustrating zotarolimus blood concentrations as a function of time and coating formulation in accordance with one embodiment of the invention; and

FIG. 8 is a graph illustrating zotarolimus blood concentrations plotted as a function of time per each dose and coating formulation using a deconvolution and then convolution model in accordance with one embodiment of the invention;

## DETAILED DESCRIPTION

Reference will now be made in detail to the various aspects of the disclosed subject matter. The method and corresponding steps of the invention will be described in conjunction with the detailed description of the device, the figures and examples provided herein.

The devices and methods presented can be used for treating the lumen of a patient. In particular, the invention is particularly suited for treatment of the cardiovascular system of a patient, such as performance of angioplasty and delivery of a balloon expandable medical device, such as a stent, filter and coil.

A balloon catheter is disclosed herein for delivering a therapeutic agent to the vasculature of a patient and also other target tissues. The balloon catheter has an elongate member having a proximal end, a distal end, and a lumen therebetween. An expandable balloon is disposed near the distal end of the elongate tubular member. A coating is applied to at least a portion of the balloon catheter, the coating including an cytostatic therapeutic agent, an excipient and a plasticizer. The term "cystostatic" as used herein refers to a drug or compound which possesses the dual properties of mitigating cell proliferation and allowing cell migration. The drug delivery balloon and its coating is configured so that at least 30% of the coating transfers from the balloon surface within two minutes after inflation of the balloon. Preferably, however, at least 90% of the coating transfers from the balloon surface within two minutes after inflation of the balloon and more preferably at least 50 to at least 75 percent of the coating transfers from the balloon surface within two minutes after inflation of the balloon.

The coating of the drug balloon catheter produces a pharmacokinetic profile that provides the therapeutic agent to the vasculature or target tissue in a sufficient and effective concentration. This concentration is effective in preventing or inhibiting restenosis. Indeed, the resulting pK profile or decline in tissue concentration with time can provide the therapeutic agent at a concentration necessary to prevent or inhibit restenosis. Pharmacokinetics includes the study of the mechanisms of absorption and distribution of an administered drug, the rate at which a drug action begins and the duration of the effect, the chemical changes of the substance in the body (e.g. by enzymes) and the effects and routes of excretion of the metabolites of the drug. Pharmacokinetic analysis is performed by noncompartmental (model independent) or compartmental methods. Noncompartmental methods estimate the exposure to a drug by estimating the area under the curve of a concentration-time graph. Compartmental methods estimate the concentration-time graph using kinetic models. Compartment-free methods are often more versatile in that they do not assume any specific compartmental model and produce accurate results also acceptable for bioequivalence studies.

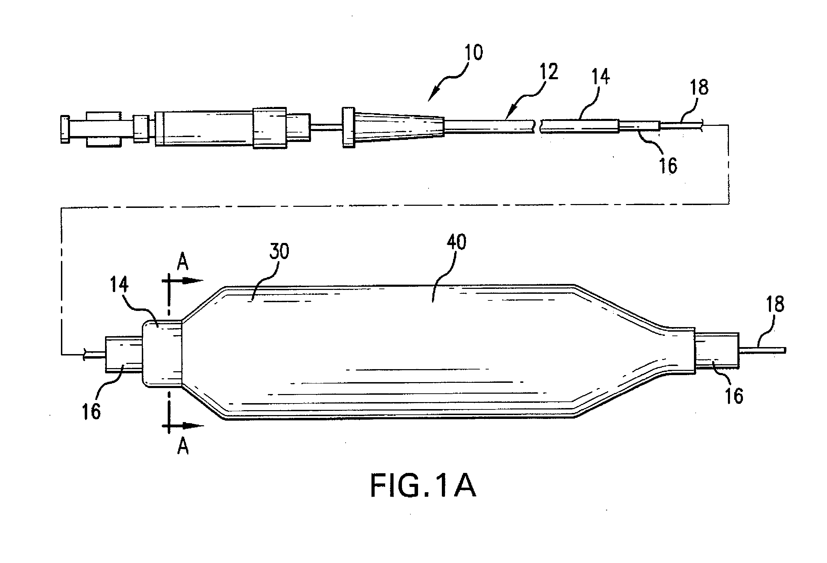

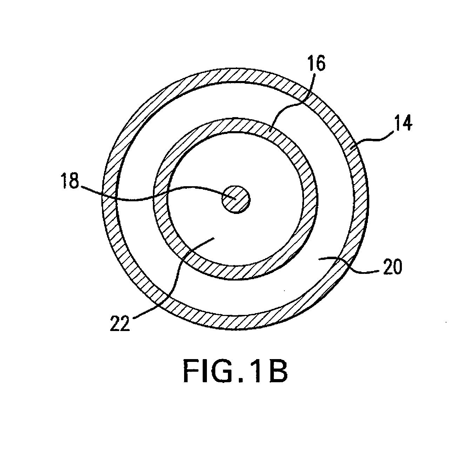

A balloon catheter device is shown schematically in Figs. 1a and 1b. As shown in Figs. 1a and 1b, the balloon catheter device 10 generally includes an elongated catheter shaft 12 having a proximal end and having a distal end and an expandable balloon 30 located proximate to the distal end of the catheter shaft. The expandable balloon has an outer surface and an inner surface disposed at the distal end portion of the catheter shaft. The coating 40 is applied to at least one portion of the balloon catheter, the coating including a cytostatic therapeutic agent and an excipient. The coating also includes a plasticizer. The coating is configured such that at least 30% of the coating transfers from the balloon surface within two minutes after inflation of the balloon. The coating is applied to at least one portion of the outer surface of the balloon catheter.

The elongated catheter shaft 12 comprises an outer tubular member 14 and an inner tubular member 16. The outer tubular member 14 defines an inflation lumen 20 that can be disposed between the proximal end portion and the distal end portion of the catheter shaft 12. Specifically, as illustrated in Fig. 1b, the coaxial relationship between the inner tubular member 16 and the outer tubular member 14 defines an annular inflation lumen 20. The expandable member 30 is placed in fluid communication with the inflation lumen 20. The inflation lumen can supply fluid under pressure, and establish negative pressure to the expandable member. The expandable member 30 can thus be inflated and deflated. The elongated catheter is sized and configured for delivery through a tortuous anatomy, and can further include a guidewire lumen 22 that permits it to be delivered over a guidewire 18. As illustrated in Fig. 1b, the inner tubular member 16 defines the guidewire lumen 22 for the guidewire 18. Although Figs. 1 and 1b illustrate the guidewire lumen as having an over-the-wire (OTW) construction, the guidewire lumen can be configured as a rapid-exchange (RX) construction, as is well known in the art.

In accordance with the invention, a drug delivery balloon having a coating including an cytostatic therapeutic agent, an excipient and a plasticizer is configured such that at least 30% of the coating transfers from the balloon surface within two minutes after inflation of the balloon. Furthermore, the drug delivery balloon of the present invention contains a coating and the resulting pK profile based upon the coating demonstrates that a sufficient therapeutic agent concentration is deposited at the tissue site of interest. The resulting PK profile itself, or decline in tissue concentration with time, provides the therapeutic agent at a concentration necessary to inhibit and/or prevent restenosis.

The coating of the drug delivery balloon of the present invention is configured such that at least 30% of the coating transfers from the balloon surface within two minutes after inflation of the balloon. In accordance with the invention, the coating can be applied to the medical device by processes such as dip-coating, pipette coating, syringe coating, air assisted spraying, electrostatic spraying, piezoelectric spraying, electrospinning, direct fluid application, or other means as known to those skilled in the art. The coating can be applied over at least a portion or the entirety of the balloon or medical device. By way of example, and not limitation, certain coating processes that can be used with the instant invention are described in U.S. Patent No. 6, 669, 980 to Hansen; U.S. Patent No. 7, 241, 344 to Worsham; and U.S. Publication No. 20040234748 to Stenzel. In accordance with one embodiment of the invention, the medical device is a balloon catheter and the coating can be applied to either a folded or inflated balloon. Furthermore, the coating can be directly applied into the folds of the folded balloons. The coating characteristics are affected by process variables. For example, for dip-coating process, coating quality and thickness can vary as an effect of variables such as number, rate, and depth of dips along with drying time and temperature. In accordance with a preferred embodiment, the coating is applied via a Sonotek piezoelectric spray coater modified to fully inflate and rotate the angioplasty balloon subassemblies.

In accordance with a preferred embodiment, after coating, the subassembly is baked dry, and then tri-folded and heat set to a low profile of 0.053" or less. An optional bare metal stent can then be crimped onto the balloon using an icy hot or other process. The balloon is then sheathed and a full-length catheter is heat or laser bonded before being packaged and either EtO or e-beam sterilized.

The coating is thus designed to wet and/or swell during folded balloon delivery and tracking. During one or more inflations, and contact with the vessel wall for less than two minutes, preferably less than one minute, depending on the particular type of cytostatic drug coated on the balloon surface, at least 30% of the coating transfers from the balloon surface. The fast dissolution of the coating results in effective release of the therapeutic agent to the vasculature or target tissue site of interest. Following delivery of the therapeutic agent to the site of interest, the balloon is rapidly deflated and removed.

In accordance with the invention, excipients are utilized together with the therapeutic agent in the coating at ratios ranging from 1:20 to 20:1 excipient:drug by weight, preferably from 1:10 to 2:1, more preferably from 1:3 to 1:1. The excipients provide improved release from the balloon, improved tissue uptake and retention, enhanced adhesion, and/or product stability and shelf life. In the absence of an excipient, a pure drug would be expected to produce the lowest coating profile or thickness at the same dosage and coating uniformity.

In accordance with a preferred embodiment, the excipients of the present invention are water soluble. The excipients can include non-ionic hydrophilic polymers. Non-ionic hydrophilic polymers include, but are not limited to, poly(vinyl pyrrolidone) (PVP, Plasdone, povidone), silk-elastin like polymer, poly(vinyl alcohol), poly(ethylene glycol) (PEG), pluronics (PEO-PPO-PEO), poly(vinyl acetate), poly(ethylene oxide) (PEO), PVP-vinyl acetate (copovidone), PEG phospholipids, and polysorbates such as polysorbate 20 (Tween 20). Preferably, the molecular weight of non-ionic hydrophilic polymers can be less than 50 kDa for fast solubility. The excipient can also include fatty acids. Further, the excipient can be a lubricious material which improves spreading and uniformity of coating.

In addition, a plasticizer is added to the binder to keep it soft and pliable. Plasticizers can allow for greater coating flexibility and elongation to prevent coating cracking during inflation or brittleness. Plasticizers include, but are not limited to, glycerol, ethanol, dimethylsulfoxide, triethyl citrate, tributyl citrate, acetyl tributyl citrate, acetyl triethyl citrate, dibutyl phthalate, dibutyl sebacate, dimethyl phthalate, triacetin, polyethylene glycol, propylene glycol, 2-pyrridone, benzyl alcohol, N-methylpyrrolidone, Cremophor EL, sucrose, sorbitol, water, and combinations thereof. Preferably, a biocompatible plasticizer is used.

In accordance with yet another embodiment, anti-coagulants can be used as a binder for the particles. For example, heparin based polysaccharides can provide a minimally thrombogenic surface to prevent blood clotting on the balloon surface or minimize platelet activation induced by the procedure. Heparin based polysaccharides include, but are not limited to, heparin, heparin sulfate, heparin disaccharides, heparin fraction 1, heparin fraction 2, low molecular weight heparin, heparin ammonium, heparin calcium, heparin lithium, heparin lithium, and heparin zinc lithium. Low molecular weight heparin includes centaxarin, periodate-oxidized heparin, heparin sodium end-amidated, heparin sodium, and nitrous acid delaminated.

In accordance with a preferred embodiment of the invention, the excipient possesses a mucoadhesive property. This mucoadhesive property of the binder will lead to longer drug retention within the coating adhered to the vessel wall. In particular, charged excipients such as chitosan, polyacrylic acid, polyglutamic acid, some polysaccharides (e.g. carboxymethylcellulose (CMC), sodium hyaluronate, sodium alginate) and some non-ionic hydrophilic polymers exhibit mucoadhesive properties. Any above carboxylated materials can also be lightly activated with esters such as nitrophenolate or NHS-esters (N-hydroxy succinimide) for increased mucoadhesiveness. Alternatively, any above materials can be lightly thiolated for increased mucoadhesiveness and continued solubility.

Additionally or alternatively, the excipient is or includes a contrast agent, including but not limited to, Iopromide (Ultravist), Ioxaglate (Hexabrix), Ioversol (Optiray), Iopamidol (Isovue), Diatrixoate (Conray), Iodixanol (Visipque), and Iotrolan. At an intermediate coating thickness, a lower molecular weight (< 1 kDa) hydrophilic contrast agent such as Iopromide (Ultravist) would enable faster therapeutic release and a slightly higher viscous coating of the vessel wall as compared with drug alone.

In accordance with one embodiment, polyvinylpyrrolidone (PVP) having a $M_W$ of 100 kDa or less would be expected to provide a means of faster coating release and increased mucoadhesiveness against the vessel wall. The swellable nature of this non-ionic hydrophilic polymer when hydrated and especially when plasticized with glycerol produces a thicker and toughened coating.

The cytostatic therapeutic agent is present in the coating in a therapeutic amount.

For example and not limitation, the coating can include a therapeutic agent in addition to the cytostatic drug. In this regard, the therapeutic agent can include anti-proliferative, anti-inflammatory, antineoplastic, antiplatelet, anticoagulant, anti-fibrin, antithrombotic, antimitotic, antibiotic, antiallergic and antioxidant compounds, HMG-CoA reductase inhibitors, and peroxisome proliferator-activated receptor a (PPAR $\alpha$) agonists such as fenofibrates (clofibrate, ciprofibrate, benzafibrate, and Tricor and Trilipix ABT-335). Thus, the therapeutic agent can be, again without limitation, a synthetic inorganic or organic compound, a protein, a peptide, a polysaccharides and other sugars, a lipid, DNA and RNA nucleic acid sequences, an antisense oligonucleotide, an antibodies, a receptor ligands, an enzyme, an adhesion peptide, a blood clot agent including streptokinase and tissue plasminogen activator, an antigen, a hormone, a growth factor, a ribozyme, and a retroviral vector.

As illustrated in Example 2, described in detail below, balloons coated with everolimus formulations were evaluated. The three formulations are summarized in Table 1 and the experimental procedures and details are described in Example 2, below. In brief, balloon expansion was performed in healthy domestic porcine coronary arteries. The balloons were maintained expanded in position for 30 seconds. The animals were sacrificed after 24 hours and 72 hours after balloon dilation and drug delivery after which the concentration of the everolimus in the arterial tissue at the expansion sites was measured.

Table 1: Summary of Everolimus DC Balloons Evaluated

| Balloon | Formulation | Dosage of Therapeutic Agent on Balloon ($\mu$g/balloon) |
|---|---|---|
| 1 | Everolimus alone plus bare metal stent (BMS) | 1600 |
| 2 | Everolimus:Ultravist Contrast Agent [1.95:1 (w/w) ratio] plus bare metal stent (BMS) | 1250 |
| 3 | Everolimus:PVP C-30:glycerol [1:1:0.4 (w/w) ratio] plus bare metal stent (BMS) | 1025 |

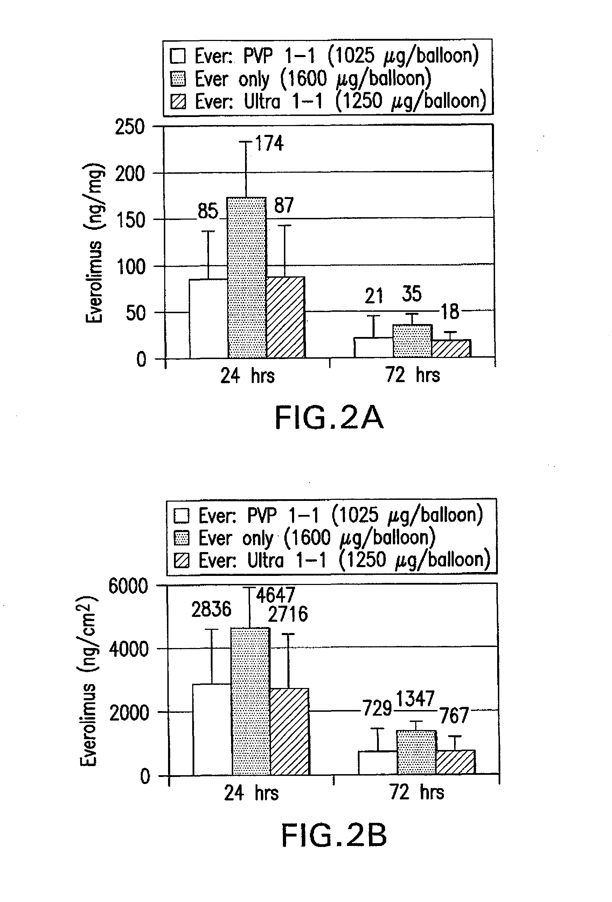

As illustrated in Figure 2a and 2b, tissue concentrations ranged from 85-174 ng/mg or 2716-4647 ng/cm$^2$ at 24 hours and 18-35 ng/mg or 729-1347 ng/cm$^2$ at 72 hours for the various coatings evaluated. There was no significant difference observed among the various formulations at either time-point (One-way Anova, $p > 0.05$). Accordingly, based on the values observed at 24 hours and 72 hours it is expected that everolimus concentrations will persist in the tissue post delivery, up to 7 days post delivery or longer. With these two time-points, and assuming a one component model with an exponential decay, tissue half lives for the drug can be calculated.

The equation for the exponential decay model is:

$$C_T = C_0 \exp^{-kT} \qquad \textbf{(EQ. 1)}$$

where $C_T$ is the concentration at time T, $C_0$ is the concentration at time zero, and k is the decay constant. The results of the pK data assuming a one component model with an exponential decay are summarized in Table 2.

Table 2. Everolimus pK Data Fitted to Simple Exponential Decay Model

| Formulation | Based on Tissue Concentration | | Based on Arterial Area | |
|---|---|---|---|---|
| | $C_0$ (ng/mg) | k and $T_{1/2}$ | $C_0$ (ng/cm$^2$) | k and $T_{1/2}$ |
| Ever/PVP 1025 ug/balloon | 171 | 2.9E-2 hr$^{-1}$, 24 hr | 5590 | 2.8E-2 hr$^1$, 25 hr |
| Ever Only 1600 ug/balloon | 388 | 3.3E-2hr$^{-1}$, 21 hr | 8630 | 2.6E-2 hr$^1$, 27 hr |
| Ever/Ultra 1250 ug/balloon | 191 | 3.3E-2 hr$^{-1}$, 21 hr | 5110 | 2.6E-2 hr$^1$, 27 hr |

In Table 2, the initial concentration ($C_0$) values of everolimus, concentration in the tissue are indicative of the concentrations at T=0. However, the actual $C_0$ tissue concentrations are higher. This is because at short times the transferred coating system is heterogeneous and the entire drug has not actually or totally dissolved into the tissue. Indeed, some of the drug is still present as films or particulates pressed onto or into the vessel wall. The maximum tissue concentrations appear to be more a function of the amount of drug on the balloon rather than which of these three formulations was used. As illustrated in Table 2 above, the decay constants (k) are quite similar, thus indicating that the decrease in drug concentration over time was not dependent on which of the three formulations was used. This indicates that once the drug has dissolved into the tissue, the pharmokinetic profile property of the drug is primarily dependent on the chemical characteristics of the drug itself.

For comparative purposes, Table 3 tabulates the concentration of everolimus in tissue using either the everolimus eluting stent system (XIENCE) and the everolimus eluting balloon of the present invention. The results from the pharmacokinetic studies for both the everolimus eluting stent system and everolimus eluting balloon of the present invention are presented as tissue concentrations in Table 3.

**Table 3. Comparison of Drug-Coated Balloon and Drug-Eluting Stent XIENCE Everolimus Tissue Concentrations**

| Timepoint (Days) | Everolimus XIENCE Tissue Concentration (ng/mg, mean) | Everolimus DCB Tissue Concentrations (ng/mg, ranges) |
|---|---|---|
| 1 | 2.2-3.2 | 85-174 |
| 3 | 1.5-1.8 | 18-35 |
| 7 | 1.6 | NA |
| 14 | 0.8-1.6 | NA |

At the 1 day and 3 day time-points, the drug-coated balloon tissue concentrations are 10-80 times higher than those seen with drug-eluting stent. However, it is not precisely known how the drug tissue concentration profile relates to efficacy against restenosis in the clinic. Certainly, however, if the tissue concentrations match, or are greater than the drug-eluting stent at time-points out to 7, 14, and perhaps 28 days, then it is reasonable to state that the drug-coated balloon is as effective in reducing restenosis.

In accordance with a further embodiment, tissue concentrations for the drug-coated balloon were extrapolated out to longer time points with the simple exponential model. Although the exponential model is a substantial oversimplification, it can provide order of magnitude estimates. The results of extrapolation to longer time points are summarized in Table 4.

Table 4. Extrapolation of DC Balloon Everolimus Tissue Concentrations

| Timepoint (Days) | Ever/PVP 1025 ug/balloon Tissue Conc. (ng/mg) | Ever Only 1600 ug/balloon Tissue Conc. (ng/mg) | Ever/Ultra 1250 ug/balloon Tissue Conc. (ng/mg) |
|---|---|---|---|
| 1 | 85 | 176 | 87 |
| 3 | 21 | 36 | 18 |
| 7 | 1.3 | 1.5 | 0.75 |
| 14 | 0.01 | 0.006 | 0.003 |

As illustrated in Table 4, the tissue concentrations for the everolimus coated balloons are all equal to or greater than the tissue concentrations from the everolimus drug-eluting sent (XIENCE) out to 7 days. However, according to the model, after seven days, the tissue concentrations drop below the drug-eluting stent. However, based on various trials which have indicated that a fast drug release is highly effective, it is reasoned that an effective tissue concentration out to seven days should be adequate in inhibiting restenosis. The trials which have indicated that a fast drug release is highly effective include, but are not limited to, the sirolimus-eluting stent (Cypher FIM trial), where the quick drug release arm was highly effective. The sirolimus eluting stent had roughly 89% drug release at four days with 98% release at 15 days and was highly effective.

Based on the input parameters in Table 5 below, a pK mathematical fit was performed to further extrapolate information from the pK porcine data. This elimination model assumed that mass of drug was eliminated as a function of disintegration and dissolution and written mathematically as

$$\frac{dm}{dt} = -K1m - K21A \qquad \textbf{(EQ. 2)}$$

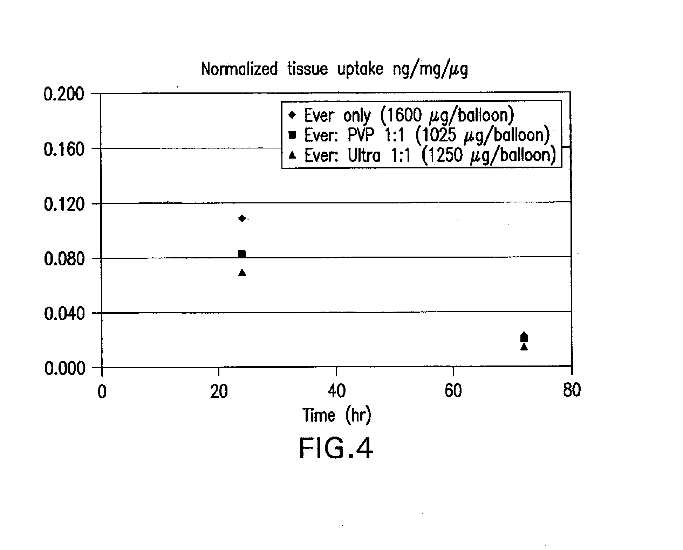

Where m = mass, K1 and K21 are constants, A = surface area, and initial boundary conditions of m (t=0) = rM0. Interpretations on normalizing pK data as a function of balloon dose are illustrated in Figure 4.

Table 5. Input parameters and extracted fitted parameters for pK Fit

| Bi-exponent constants | Ever-only | Ever-ultra | Ever-PVP |
|---|---|---|---|
| K1 | 1 | 1 | 1 |

(continued)

| Bi-exponent constants | Ever-only | Ever-ultra | Ever-PVP |
|---|---|---|---|
| K21 | 0.018 | 0.018 | 0.018 |
| r | 1 | 0.5 | 0.42 |
| Initial dose ratio | 0.18 | 0.11 | 0.13 |
| M0 | 1600 | 1250 | 1025 |
| Total mass/balloon | 1600 | 2500 | 2460 |
| Thickness ratio initial dose | .18 | .22 | .31 |

In comparing the results from the mathematical fit model of Equation 2, as summarized in Table 5, the drug delivery balloon of the present invention having a coating including an cytostatic therapeutic agent and excipient provides for an effective transfer of coating from the balloon surface within a short time period. As illustrated in Table 5, the rate constants (K1, K21) for elimination by physical dislodgement and by dissolution/diffusion is similar among different formulations. Furthermore, the thinner and higher concentration coating may have a more effective initial drug uptake. Indeed, the initial mass transfer into the vessel wall is dependent on the coating thickness (0.18, 0.22, 0.31). A brittle coating will have less initial mass transfer to the wall due to greater sensitivity to mechanical perturbation (0.2 vs. 0.3). The everolimus-PVP coating increases toughness and hence obtains higher initial transfer to the tissue when compared to the everolimus-Ultravist coating. According to results of mathematical fit, the thicker coatings may have higher variability in tissue uptake. Further, a coating with a therapeutic agent only behaves similar to coating including both a therapeutic agent and an excipient in both mechanical integrity and local pK.

Figure 4 illustrates interpretations on normalizing pK data as a function of balloon dose. In this example, ng/mg/ug refers to ng released drug per mg tissue per ug original drug dose. As illustrated in Figure 4, the initial variation of drug tissue uptake among the different formulations decreases with time. Everolimus only had the highest 110 (ng drug/mg tissue/ug original dose) as compared to everolimus with Ultravist at 75 (ng drug/mg tissue/ug original dose) while everolimus with PVP exhibited an intermediate value. However, at the end of 72 hours post drug delivery, all three formulations were approximately 20 (ng drug/mg tissue/ug original dose). Thus, tissue uptake at longer times may be a stronger function of diffusion rather than dislodgement.

In accordance with the invention, the everolimus has a concentration between 88 to 1500 ug/cm² balloon. In accordance with the invention, the released concentration of everolimus in the tissue decreases by more than 50% after about 72 hours post inflation of the balloon. In accordance with yet another embodiment, the released concentration of everolimus in the tissue decreases by more than 90% after about 72 hours post inflation of the balloon. Preferably, the everolimus concentration in the blood system does not exceed 250 ng/ml 24 hours post balloon inflation. In accordance with the invention, the everolimus concentration in the blood system does not exceed 179 ng/ml 24 hours post balloon inflation.

As illustrated in Example 3, described in detail below, balloons coated with zotarolimus formulations were evaluated. The six formulations are summarized in Table 6 and the experimental procedures and details are described in Example 3, below. In brief, balloon expansion was performed in healthy domestic porcine coronary arteries. The balloons were maintained expanded in position for 30 seconds. The animals were sacrificed after 30 minutes, 1 day (zotarolimus alone), and 7 days after delivery and the concentration of the zotarolimus in the arterial tissue at the expansion sites was measured.

Table 6. Summary of Zotarolimus DC Balloons Evaluated

| Balloon | Formulation | Dosage of Therapeutic Agent on Balloon ($\mu$g/cm²) |
|---|---|---|
| 1 | Zotarolimus alone plus bare metal stent (BMS) | 88 $\mu$g/cm² |
| 2 | Zotarolimus alone bare metal stent (BMS) | 570 $\mu$g/cm² |
| 3 | Zotarolimus:Ultravist Contrast Agent [1.95:1 (w/w) ratio] plus bare metal stent (BMS) | 88 $\mu$g/cm² |
| 4 | Zotarolimus:PVP:Glycerol [2:1:0.4 (w/w) ratio] | 88 $\mu$g/cm² |
| 5 | Zotarolimus:PVP:Glycerol [2:1:0.4 (w/w) ratio] plus bare metal stent (BMS) | 15 $\mu$g/cm² |

(continued)

| Balloon | Formulation | Dosage of Therapeutic Agent on Balloon ($\mu$g/cm$^2$) |
|---|---|---|
| 6 | Zotarolimus:PVP:Glycerol [2:1:0.4 (w/w) ratio] | 15 $\mu$g/cm$^2$ |

Table 7. Summary of Zotarolimus blood pharmacokinetic parameters

| Study Arm | C$_{max}$ (ng/mL) | C$_{max}$/dose (ng/mL/ug) | T$_{max}$ (h) | AUC$_5$ (ng/mL*h) | AUC$_5$ / dose (ng/mL/ug*h) |
|---|---|---|---|---|---|
| Zot only 570ug/cm$^2$ (3x DCB) | 39.4 $\pm$ 11.1* | 0.020 $\pm$ 0.01* | 2* | NA | NA |
| Zot only 88ug/cm$^2$ (3x DCB) | 9.8 $\pm$ 2.6* | 0.028 $\pm$ 0.01* | 2* | NA | NA |
| Zot-Ultra 1.95-1 88ug/cm$^2$ (3x DCB) | 10.1 $\pm$ 3.2 | 0.031 $\pm$ 0.01 | 3 | 31.7 $\pm$ 10.9 | 0.085 $\pm$ 0.03 |
| Zot-PVP-gly 2-1-0.4 88ug/cm$^2$ (5x DCB) | 19.2 $\pm$ 3.1 | 0.030 $\pm$ 0.01 | 2 | 62.3 $\pm$ 5.4 | 0.082 $\pm$ 0.01 |
| Zot-PVP-gly 2-1-0.4 15ug/cm$^2$ (5x DCB) | 2.1 $\pm$ 0.8 | 0.025 $\pm$ 0.01 | 1 | 6.9 $\pm$ 1.5 | 0.070 $\pm$ 0.01 |

Blood zotarolimus concentrations increased as a function of time with Tmax ranging from 1-3 hours and Cmax from 2.1-39.4 ng/mL as a function of coating formulation as summarized in Table 7. The blood concentrations appeared to exist more as a function of dose than excipient once the values were normalized. This trend indicates that excipients may serve more as both a binder and hydrophilic spacer than drug solubilizer.

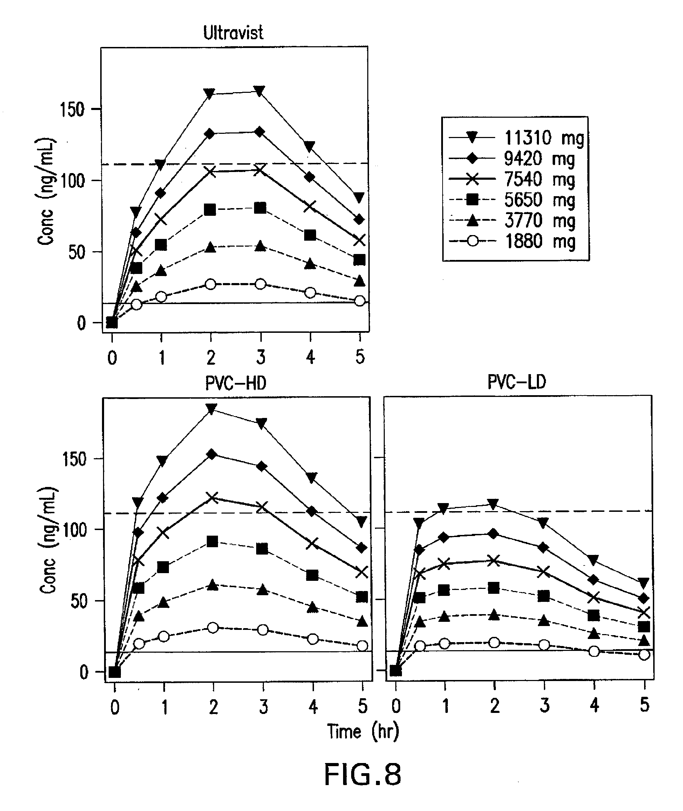

The data of zotarolimus concentration in the arterial tissue can be further modeled using a pK deconvolution/ convolution model to predict blood concentration as function of total dose per each formulation as shown in Figure 8. As illustrated in Fig. 8, the zotarolimus blood concentrations predicted as a function of dose (1880 to 11310 mg) and coating formulation using a deconvolution and then convolution model.

For comparative purposes, Table 8 tabulates the concentration of zotarolimus in tissue using either the zotarolimus eluting stent system (Endeavor) and the zotarolimus eluting balloon of the present invention. The results from the pharmacokinetic studies for both the zotarolimus eluting stent system and zotarolimus eluting balloon of the present invention are presented as tissue concentrations in Table 8.

**Table 8. Comparison of Drug-Coated Balloon and Drug-Coated Stent (Endeavor) Zotarolimus Tissue Concentrations**

| Timepoint (Days) | Zotarolimus Endeavor Tissue Concentration (ng/mg, mean) | Zotarolimus DCB Tissue Concentrations (ng/mg, ranges) |
|---|---|---|
| 1 | 23.92 | 11-993 |
| 7 | 13.19 | 0.03-22 |
| 14 | 4.31 | NA |

At the 1 day timepoint, the drug-coated balloon tissue concentrations are 1-40 times higher than those seen with drug-eluting stent. However, it is not precisely known how the drug tissue concentration profile relates to efficacy against restenosis in the clinic. Certainly, however, if the tissue concentrations match, or are greater than the drug-eluting stent at time-points out to 7 and perhaps 14 days, then it is reasonable to state that the drug-coated balloon is as effective.

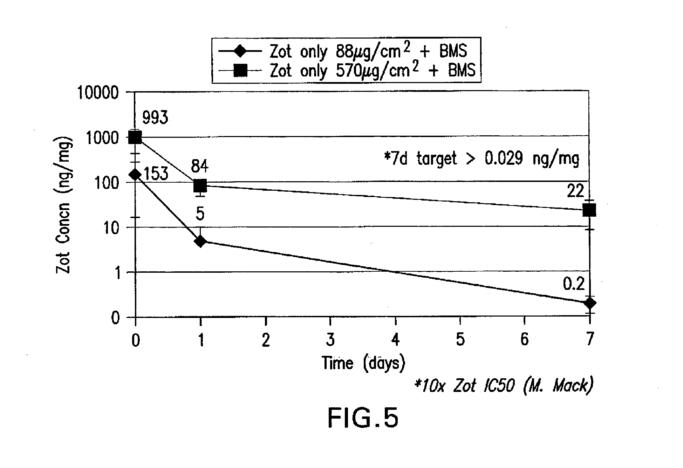

Assuming a one component model with an exponential decay, tissue half lives for the drug can be calculated using the zotarolimus tissue concentration depicted in Figure 5. At the 30 minute timepoint, the tissue concentrations are high and can represent a heterogeneous state where discrete pieces of drug may be embedded in the vessel wall. Hence, for the purposes of calculating an exponential decay and a highlife, we use only the data points at 1 and 7 days. From these values we arrive at the data shown in Table 9.

Table 9. Exponential fit model for 1 & 7 day time-points of Zotarolimus only pK

| Zotarolimus Dose (ug/cm$^2$) | k(hr$^{-1}$) | Half Life (hours) | Calculated C$_0$ (ng/mg) |
|---|---|---|---|
| 88 | 0.54 | 31 | 8.6 |
| 570 | 0.22 | 75 | 105 |

This fit provides an estimate for the tissue half-life to be in the range of 31 to 75 hours. The tissue half-life is probably dose dependent but the doses of interest for drug coated balloons lie largely in the range of 88 to 570 ug/cm2. In Table 9, the initial concentration (C$_0$) values of zotarolimus concentration in the tissue are indicative of the concentrations at T=0. However, the calculated value of Co is much lower than the concentration measured at 30 minutes, therefore, indicating the lack of fit and possible presence of solid drug at short time points.

In accordance with a further embodiment, tissue concentrations for the drug-coated balloon were extrapolated out to longer time points with a non-linear fit model:

$$\frac{1}{Z(t)} = a + bt^{1.5}$$

Although the non-linear fit model is a substantial oversimplification, it can provide order of magnitude estimates. The results of extrapolation of zotarolimus tissue concentrations to longer time points are summarized in Table 10.

Table 10. Extrapolation of DC Balloon Zotarolimus Tissue Concentrations

| Timepoint (Days) | Zotarolimus only 88 ug/cm$^2$ dose (ng/mg) | Zotarolimus only 88 ug/cm$^2$ dose fit (a = 0.005952, b = 0.1941) (ng/mg) | Zotarolimus only 540 ug/cm$^2$ dose (ng/mg) | Zotarolimus only 540 ug/cm$^2$ dose fit (a = 0.0009903, b= 0.005652) (ng/mg) |
|---|---|---|---|---|
| 0.02083 | 153 | 153 | 993 | 1117 |
| 1 | 5 | 5 | 84 | 374 |
| 7 | 0.2 | 0.28 | 22 | 29.5 |
| 14 | NA | 0.098 | NA | 3.4 |

As illustrated in Table 10, the tissue concentrations for the zotarolimus coated balloons are all equal to or greater than the tissue concentrations from the zotarolimus drug-eluting sent (Endeavor) out to 7 days. However, according to the parametric model, after seven days, the tissue concentrations drop below the drug-eluting stent. However, based on various trials which have indicated that a fast drug release is highly effective, it is reasoned that an effective tissue concentration out to seven days should be adequate. The trials which have indicated that a fast drug release is highly effective include, but are not limited to, the sirolimus-eluting stent (Cypher FIM trial), where the quick drug release arm was highly effective. The sirolimus eluting stent had roughly 89% drug release at four days with 98% release at 15 days and was highly effective.

In accordance with the invention, the zotarolimus has a concentration between 15 to 600 ug/cm2. In accordance with the invention, the released concentration of zotarolimus in the tissue decreases by more than 50% after about 72 hours post inflation of the balloon. In accordance with yet another embodiment, the released concentration of zotarolimus in the tissue decreases by more than 90% after about 72 hours post inflation of the balloon. Preferably, the zotarolimus concentration in the blood system does not exceed 232 ng/ml 5 hours post balloon inflation. In accordance with the invention, the zotarolimus concentration in the blood system does not exceed 111 ng/ml 2 hours post balloon inflation.

In accordance with the drug delivery device of the present invention, the use of zotarolimus coated balloons configured to transfer at least 30% of the coating from the balloon surface within two minutes after inflation of the balloon is effective. In accordance with the present invention, tissue concentrations and blood concentrations increased as a function of larger zotarolimus dosage. Furthermore, excipients, such as Ultravist and PVP-glycerol, increased acute drug uptake and tissue concentrations compared with zotarolimus only coatings in conjunction with bare metal stent implantation. In fact, less acute drug uptake resulted from a drug-coated balloon only versus a drug-coated balloon and bare metal stent system.

In accordance with the present invention, the cytostatic coating provides a pharmacokinetic profile post bolus delivery from a drug coated balloon that result in an efficacious response in reduction of restenosis after balloon and bare metal stent trauma. The drug delivery balloon of the present invention having a coating including an cytostatic therapeutic agent and an excipient provides for a bolus release of the cytostatic therapeutic agent with an inflation time of two minutes or less. At least thirty percent of the coating transfers from the balloon surface within two minutes after inflation of the balloon.

In accordance with one embodiment, $C_{max}$, or the maximum tissue concentration, occurs in the time frame of 1-60 minutes, preferably between 10-2000 ng/mg [ng drug/mg tissue], and more preferably between 10-250ng/mg [ng drug/mg tissue]. In accordance with the invention, in order to achieve the desired pK profile, this $C_{max}$ must be at least 10 ng/mg. Preferably, the concentration of cytostatic drugs in the blood system does not exceed 232 ng/ml 5 hours post inflation. More preferably, however, the concentration of cytostatic drugs does not exceed 111 ng/ml 2 hours post inflation.

In a further embodiment, the drug delivery balloon produces a pK profile with a drug tissue concentration half life in the range of about 10 to about 100 hours. However, depending on the drug used the half life can range from about 18 to about 48 hours or about 24 to 96 hours. Furthermore, this desired pK profile should be such that at one day, the tissue concentration of the therapeutic agent is in the range of 10-1000 ng/mg, preferably from 10-250 ng/mg and the seven day tissue concentration of the therapeutic agent is greater than 29 nM.

In accordance with the present invention, the coating provides a controlled release of the cytostatic drug over a period of at least 72 hours post inflation of the balloon. However, in a preferred embodiment, the coating provides a controlled release of the cytostatic drug over a period of at least one week. Moreover, the coating can provide a controlled release of the cytostatic drug over a period of at least two weeks.

The drug delivery balloon of the present invention is effective in that the therapeutic agent is retained in the vessel wall due to the permeation/uptake of drug. When compared to the drug-eluting stent, the drug delivery balloon of the present invention occupies at least 75% of the arterial wall area. Hence the drug tissue concentration, on the average, is three times more uniform with respect to the arterial surface with a drug-coated balloon than with a drug-eluting stent. Furthermore, the pK profile of the cytostatic coating of the present invention within arterial tissue over an efficacious time period eliminates the need for a controlled release polymer coating and therefore can result in decreased polymer-induced inflammation, late stent thrombosis and other improved safety criteria.

In accordance with a further embodiment, tissue uptake of everolimus at distal region, 10-15 mm away from stenting segment, indicates that the drug coated balloon of the present invention may be beneficial for coronary artery diseases with multiple site lesions (site and regional therapy).

In accordance with a further embodiment, the drug delivery balloon is a perfusion balloon and includes a coating disposed on at least a portion of the surface of the perfusion balloon, wherein the coating includes having an cytostatic therapeutic agent, a excipient, and a plasticizer, and further wherein at least 30% of the coating transfers from the balloon surface within ten minutes after inflation of the balloon. Perfusion balloons are described in detail in U.S. Patent Nos. 5, 951, 514 to Sahota, 5, 370, 617 to Sahota, 5, 542, 925 to Orth, 5, 989, 218 to Wasicek. In another embodiment, the drug delivery balloon can be mounted on a catheter with perfusion ports as described in U.S. Patents Nos. 5, 370, 617 to Sahota, 5, 542, 925 to Orth, and 5, 989, 218 to Wasicek. Alternatively, the inflation time is 5 minutes or less or the inflation time is 2 minutes or less.

In accordance with the invention, in addition to the relatively long duration effect postulated above, a separate mechanism may be operating with the use of cytostatic type drugs. Indeed, the initial high dose delivered to the tissue may interdict pathways normally below the activity threshold of cytostatic drugs delivered from drug eluting stents. For example, with initial tissue drug concentrations in the 100+ μM range, normally inefficient cytokine blockade processes for cytostatic drugs such as blocking monocyte production of TNF-α or IL-6 can occur. This would result in reduction in neointimal formation and inflammation.

In accordance with the invention the balloon is made of a polymeric material. For example, the polymeric material utilized to form the balloon body may be may be compliant, non-compliant or semi-compliant polymeric material or polymeric blends.

In one embodiment, the polymeric material is compliant such as but not limited to a polyamide/polyether block copolymer (commonly referred to as PEBA or polyether-block-amide). Preferably, the polyamide and polyether segments of the block copolymers may be linked through amide or ester linkages. The polyamide block may be selected from various aliphatic or aromatic polyamides known in the art. Preferably, the polyamide is aliphatic. Some non-limiting examples include nylon 12, nylon 11, nylon 9, nylon 6, nylon 6/12, nylon 6/11, nylon 6/9, and nylon 6/6. Preferably, the polyamide is nylon 12. The polyether block may be selected from various polyethers known in the art. Some non-limiting examples of polyether segments include poly(tetramethylene ether), tetramethylene ether, polyethylene glycol, polypropylene glycol, poly(pentamethylene ether) and poly(hexamethylene ether). Commercially available PEBA material may also be utilized such as for example, PEBAX® materials supplied by Arkema (France). Various techniques for forming a balloon from polyamide/polyether block copolymer are known in the art. One such example is disclosed in US 6, 406, 457 to Wang.

In another embodiment, the balloon material is formed from polyamides. Preferably, the polyamide has substantial tensile strength, be resistant to pin-holing even after folding and unfolding, and be generally scratch resistant, such as those disclosed in US 6, 500, 148 to Pinchuk. Some non-limiting examples of polyamide materials suitable for the balloon include nylon 12, nylon 11, nylon 9, nylon 69 and nylon 66. Preferably, the polyamide is nylon 12.

In another embodiment, the balloon may be formed a polyurethane material, such as TECOTHANE® (Thermedics). TECOTHANE® is a thermoplastic, aromatic, polyether polyurethane synthesized from methylene disocyanate (MDI), polytetramethylene ether glycol (PTMEG) and 1, 4 butanediol chain extender. TECOTHANE® grade 1065D is presently preferred, and has a Shore durometer of 65D, an elongation at break of about 300%, and a high tensile strength at yield of about 10, 000 psi. However, other suitable grades may be used, including TECOTHANE® 1075D, having a Shore D of 75. Other suitable compliant polymeric materials include ENGAGE® (DuPont Dow Elastomers (an ethylene alpha-olefn polymer) and EXACT® (Exxon Chemical), both of which are thermoplastic polymers. Other suitable compliant materials include, but are not limited to, elastomeric silicones, latexes, and urethanes.

The compliant material may be cross linked or uncrosslinked, depending upon the balloon material and characteristics required for a particular application. The presently preferred polyurethane balloon materials are not crosslinked. However, other suitable materials, such as the polyolefinic polymers ENGAGE® and EXACT®, are preferably crosslinked. By crosslinking the balloon compliant material, the final inflated balloon size can be controlled. Conventional crosslinking techniques can be used including thermal treatment and E-beam exposure. After crosslinking, initial pressurization, expansion, and preshrinking, the balloon will thereafter expand in a controlled manner to a reproducible diameter in response to a given inflation pressure, and thereby avoid overexpanding the stent (when used in a stent delivery system) to an undesirably large diameter.

In one embodiment, the balloon is formed from a low tensile set polymer such as a silicone-polyurethane copolymer. Preferably, the silicone-polyurethane is an ether urethane and more specifically an aliphatic ether urethane such as PURSIL AL 575A and PURSIL AL10, (Polymer Technology Group), and ELAST-EON 3-70A, (Elastomedics), which are silicone polyether urethane copolymers, and more specifically, aliphatic ether urethane cosiloxanes. In an alternative embodiment, the low tensile set polymer is a diene polymer. A variety of suitable diene polymers can be used such as but not limited to an isoprene such as an AB and ABA poly(styrene-block-isoprene), a neoprene, an AB and ABA poly(styrene-block-butadiene) such as styrene butadiene styrene (SBS) and styrene butadiene rubber (SBR), and 1, 4-polybutadiene. Preferably, the diene polymer is an isoprene including isoprene copolymers and isoprene block copolymers such as poly(styrene-block-isoprene). A presently preferred isoprene is a styrene-isoprene-styrene block copolymer, such as Kraton 1161K available from Kraton, Inc. However, a variety of suitable isoprenes can be used including HT 200 available from Apex Medical, Kraton R 310 available from Kraton, and isoprene (i.e., 2-methyl-1, 3-butadiene) available from Dupont Elastomers. Neoprene grades useful in the invention include HT 501 available from Apex Medical, and neoprene (i.e., polychloroprene) available from Dupont Elastomers, including Neoprene G, W, T and A types available from Dupont Elastomers.

In accordance with another aspect of the invention, the outer surface of the balloon is modified. In this regard, the balloon surface may include a textured surface, roughened surface, voids, spines, channels, dimples, pores; or microcapsules or a combination thereof, as will be described below.

In one embodiment of the invention, the balloon is formed of a porous elastomeric material having at least one void formed in the wall of the balloon surface. For example, the entire cross section of the balloon may contain a plurality of voids. Alternatively, the plurality of void may be distributed along select portions of the balloon outer surface. For example and not limitation, the plurality of voids can be distributed only along only the working section of the balloon. The voids define an open space within the outer surface of the balloon. Preferably, the therapeutic agent is dispersed within the space defined by the plurality of voids across the cross section of the balloon outer surface.

In operation, the therapeutic agent is released or is expelled from the pores upon inflation of the balloon. In this regard, the durometer of the polymeric material of the balloon surface and in particular the depression of the void is sufficiently flexible to allow for expulsion of the therapeutic agent and/or coating contained within the plurality of voids upon inflation of the balloon. The expelled coating with therapeutic agent is released into the vessel lumen or into the tissue surrounding and contacting the inflated balloon.

In another embodiment, the balloon includes protrusions configured to contact or penetrate the arterial wall of a vessel upon inflation of the balloon. A coating containing therapeutic agent is disposed on the protrusions and when inflated the coating and/or therapeutic agent coats the tissue of the arterial wall. Alternatively, the balloon may include two concentric balloons in a nesting configuration. The coating with therapeutic agent is disposed between the two concentric balloons. Thus, the space between the two concentric balloons; one being an interior balloon and the other being an exterior balloon, acts as a reservoir. In this regard, the protrusions may include apertures for expulsion of the coating and/or therapeutic agent upon inflation of the interior and exterior concentric balloons. For example, as described in US 6, 991, 617 to Hektner. In another embodiment, the balloon may include longitudinal protrusions configured to form ridges on the balloon surface. As described in US 7, 273, 417 to Wang, the ridges can be formed of filaments spaced equidistantly apart around the circumference of the balloon. However, a larger or smaller number of ridges can alternatively be used. The longitudinal ridges can be fully or partially enveloped by the polymeric material of the balloon.

In yet another embodiment of the invention, the balloon may include microcapsules on its outer surface. In this regard, the microcapsules are configured to encompass the coating and/or therapeutic agent. Upon inflation of the balloon the microcapsules located on the surface of the balloon contact the tissue of the arterial wall. Alternatively, the microcapsules may be formed in the wall of the balloon surface. The coating and/or therapeutic agent may be released from the microcapsules by fracturing of the microcapsules and/or diffusion from the microcapsule into the arterial wall. The microcapsules may be fabricated in accordance with the methods disclosed in US 5, 1023, 402 to Dror or US 6, 129, 705 to Grantz.

In accordance with another aspect of the invention, if desired, a protective sheath may be utilized to protect the coating from being rubbed off of the balloon during the movement of the coated balloon through the body lumen. The sheath is preferably made from an elastic and resilient material which conforms to the shape of the balloon and in particular is capable of expanding upon inflation of the balloon. The sheath preferably includes apertures along a portion thereof. In operation, the inflation of the balloon causes the apertures of the sheath to widen for release of the coating and/or therapeutic agent to the tissue of the arterial wall. Preferably, the sheath has a thickness less than 10 mils. However, other thicknesses are possible.

In another embodiment, the sheath has at least one longitudinal line of weakness allowing the sheath to rupture upon inflation of the balloon and the release of the coating and/or therapeutic agent onto the tissue of the arterial wall of the vessel. Preferably, the sheath is formed from polymeric material known to be suitable for use in balloon catheters. Preferably, the sheath material is an elastomeric material which will also spring back when it splits to expose more of the body lumen to the coating. The line of weakness could be provided by various techniques known in the art. However, one non-limiting examples include perforating the sheath material. In operation, the sheath is placed over the coated balloon while in the deflated state. When the coated balloon inflated, the sheath is expanded to the extent that it exceeds its elastic limit at the line of weakness and bursts to expose and therefore release the coating and/or therapeutic agent to the tissue of the arterial wall or vessel lumen. For example, see US 5, 370, 614 to Amundson.

## EXAMPLES

The present application is further described by means of the examples, presented below. The use of such examples is illustrative only and in no way limits the scope and meaning of the invention or of any exemplified term.

EXAMPLE 1: Zotarolimus Coated Balloon

Zotarolimus was coated onto deflated 17 x 3.0 mm angioplasty balloons by syringing a solution of the drug dissolved in a mixture of Ultravist contrast agent, acetone, and ethanol onto the balloon surface. The average amount of zotarolimus coated was 662 $\mu$g per balloon. Balloon expansion was performed at 20% overstretch in porcine coronary arteries. The balloons were maintained expanded in position for 1 minute. Animals were sacrificed after 20 minutes, and the concentration of zotarolimus in the arterial tissue at the expansion sites was measured. The mean dose delivered was 6% of the total, which corresponded to a local concentration of 800 $\mu$M, at this early time point.

EXAMPLE 2: Everolimus Coated Balloon

Everolimus was coated onto inflated 3.0mm x 21 mm diameter Pebax angioplasty balloons using a custom designed Sonotek ultrasonic balloon coater. Three coating formulations were evaluated including 1) everolimus alone (1025$\mu$g/balloon); 2) everolimus with hydrophilic Ultravist contrast agent at a 1:1 (w/w) ratio; and 3) everolimus with hydrophilic non-ionic polyvinylpyrrolidone polymer (Povidone C-30) and glycerol plasticizer at a 1:1:0.4 (w/w) ratio. The dosage of therapeutic agent coated on the balloons are 1) everolimus alone (1600 $\mu$g/balloon); 2) everolimus with hydrophilic Ultravist contrast agent at a 1:1 (w/w) ratio (1250 $\mu$g/balloon); and 3) everolimus with hydrophilic non-ionic polyvinylpyrrolidone polymer (Povidone C-30) at a 1:1 (w/w) ratio (1025 $\mu$g/balloon).

The coatings were sprayed and baked dry followed by balloon folding, 3.0 mm x 18 mm Vision stent crimping, sheath placement, heat bonding to a full length catheter and hypotube seal, packaging, and ethylene oxide sterilization. Stents were delivered to either porcine LAD, LCX, or RCA coronary arteries with 30 seconds inflation times and 20% overstretch as measured by angiography. At 24 hours and 72 hours after delivery, animals were sacrificed and the artery regions from proximal, stented, distal#1, and distal#2 (15 mm away from stented region) were explanted and submitted for everolimus content measurement by HPLC or LC/MS (liquid chromatography/mass spectrometry) after tissue homogenization and extraction. The tissues were briefly homogenized in a dilution solution. After centrifugation, an aliquot of supernatant was injected onto the LC/MS column. A mobile phase gradient containing formic acid and ammonium acetate was used to elute everolimus. The everolimus concentration in tissue was then determined by the total amount in dilution solution divided by tissue weight. The limit of quantification of the method is 0.5 ng/mL. For blood, an internal standard (IS) /precipitation solution was added into a whole blood sample. After vortexing and centrifugation, supernatant from the mixture was analyzed by a HPLC column.

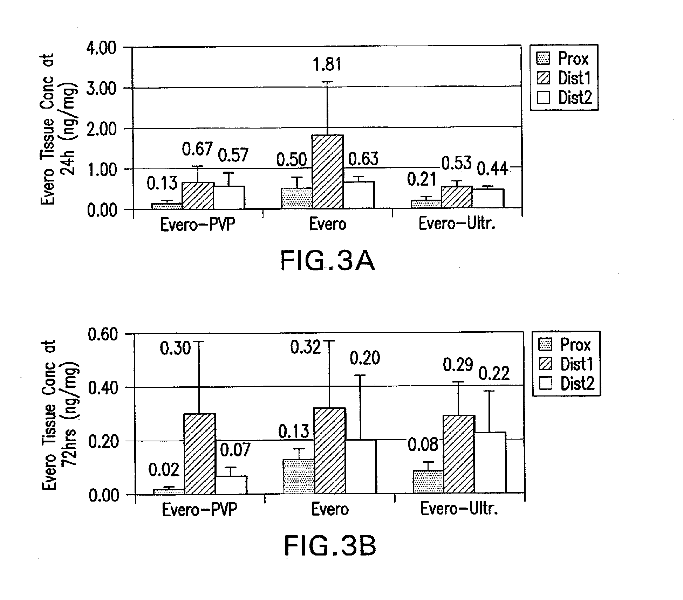

Tissue concentrations from the stented artery region are illustrated in Figure 2a and 2b. Figure 2a illustrates everolimus tissue concentrations as a function of tissue mass at 24 hours and 72 hours after delivery from everolimus coated balloons. Figure 2b illustrates everolimus tissue concentrations as a function of arterial lumen surface area at 24 hours and 72 hours after delivery from everolimus coated balloon. The balloon coating doses are also provided in the Figure legend. The everolimus tissue concentrations from proximal, distal#1 and distal#2 artery region are shown in Figures 3a and 3b. The distal#1 is 10mm away from the treated region with the distal#2 artery segment 5mm distal to the distal#1 segment. Each distal#1 and distal#2 artery region is a sample 10 mm in length. The tissue concentration of everolimus was measured at 24hrs (A) and 72hrs (B) after delivery from everolimus coated balloons.

Figure 3a illustrates tissue concentration of everoliumus at 24 hours and Figure 3b illustrates tissue concentration of everoliums at 72 hours. The values expressed in the Figures are the mean $\pm$ SD of 4 vessel segments. As illustrated in Figure 2a and 2b, tissue concentrations ranged from 85-174 ng/mg or 2716-4647 ng/cm$^2$ at 24h and 18-35 ng/mg or 729-1347 ng/cm$^2$ at 72h for the various coatings evaluated. There was no significant difference observed among the various formulations at either timepoint (One-way Anova, $p > 0.05$). Accordingly, based on the values observed at 24h and 72h it is expected that everolimus concentrations will persist in the tissue up to 7 days post delivery or more. With these two time-points, and assuming a one component model with an exponential decay, tissue half lives for the drug were be calculated, as outlined above.

EXAMPLE 3: Zotarolimus Coated Balloon

In the following experiments, zotarolimus formulations were coated onto inflated 3.0 mm x 12 mm Vision RX angioplasty balloons by air assisted spray atomization (zotarolimus only coatings at 88 ug/cm2 or 570 ug/cm2) or direct fluid volume application (zotarolimus:excipient coatings) of a solution of the drug dissolved neat in solvent or in a mixture of drug and excipient. The excipient formulations evaluated were zotarolimus-Ultravist 1.95-1 and zotarolimus-PVP-glycerol 2-1-0.4 with and without a bare metal stent and at either 88ug/cm2 or 15ug/cm2 zotarolimus dosages. Balloon expansion was performed at 20% overstretch in healthy domestic porcine coronary and/or mammary arteries. The balloons were maintained expanded in position for 30 seconds. Following balloon angioplasty, the animals were sacrificed after 30 minutes, 1 day (zotarolimus only), and 7 days and the concentration of zotarolimus in the arterial tissue at the expansion sites was measured via HPLC/LC-MS after tissue homogenization and extraction.

Treated region of interest tissue concentrations from the zotarolimus only coating porcine pK study are shown in Figure 5. Figure 5 illustrates the tissue concentrations of zotarolimus as a function of time (30 min, 1 day, 7 days) post drug coated balloon delivery in combination with a bare metal stent using a porcine model. Note the trend of larger recovery of drug tissue concentrations resulting from a higher initial (570 ug/cm$^2$ vs. 88 ug/cm$^2$ balloon zotarolimus dosage per balloon surface area. Data are expressed as the means $\pm$ stdev.

As illustrated in Figure 5, both initial concentrations decreased over the 7 day time period. They declined from an initial 993ng/mg to 22 ng/mg for 570 ug/cm$^2$ starting dose and 153ng/mg to 0.2 ng/mg for 88 ug/cm$^2$ starting drug dose. These figures represent a decrease of greater than 98%. However, for both initial 88 ug/cm$^2$ and 570 ug/ cm$^2$ drug concentrations, the final drug values from the recovered tissue are still larger than or equal to 0.03 ng/mg which is believed to be an efficacious zotarolimus tissue concentration to inhibit neointimal proliferation.

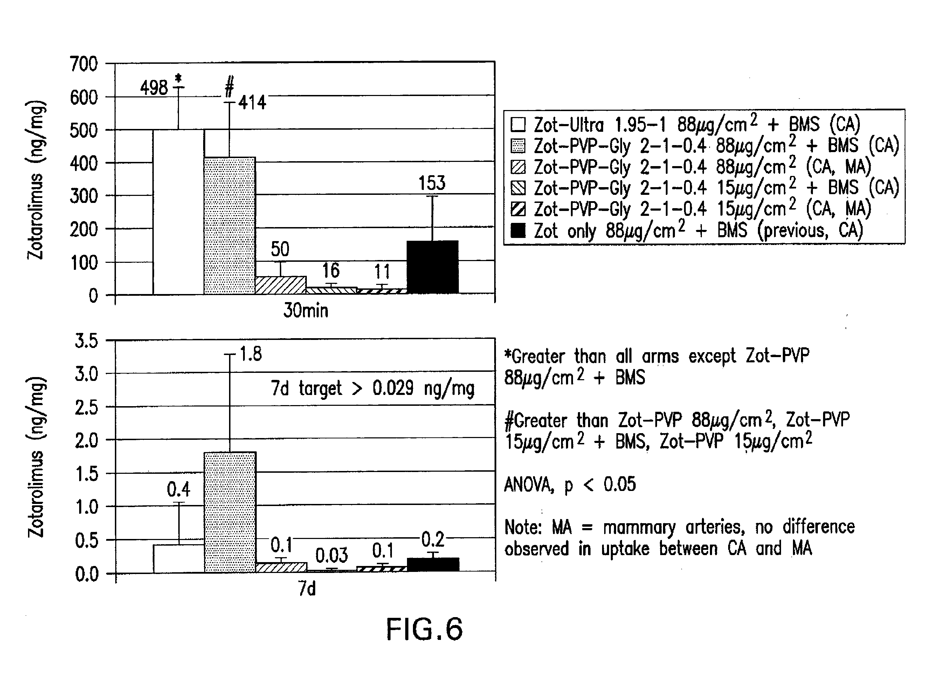

Treated region of interest tissue concentrations at 30 minutes and 7days post delivery from the zotarolimus: excipient coatings porcine PK study are shown in Figure 6. Note the similar PK behavior of high initial zotarolimus tissue concentrations that decreased over the 7 day time period to values still larger than or equal to 0.03 ng/mg which is still believed to be an efficacious tissue concentration to inhibit neointimal proliferation. From this data, by formulating with either Ultravist or PVP:glycerol excipient, significantly larger acute drug uptake in the tissue was present in the bare metal stent (BMS) combination treatment arms as compared with zotarolimus formulation without BMS at the same 88ug/cm$^2$ dose ($p < 0.05$). No significant difference was detected between the zot-ultravist or zot-PVP-glycerol with BMS tissue concentrations at either 30 minutes and 7days post delivery timepoint. Also the results demonstrated that a larger balloon zotarolimus dose per surface area resulted in higher tissue concentrations of the drug in the treated region of interest.

In Figure- 6, the treated region of interest tissue concentrations of zotarolimus:excipient coatings as a function of time (30 minutes and 7 days) post drug coated balloon delivery in a porcine model are illustrated. Note the trend of larger tissue concentrations resulting from a higher balloon zotarolimus dosage per surface area. Data are expressed as the means $\pm$ stdev.

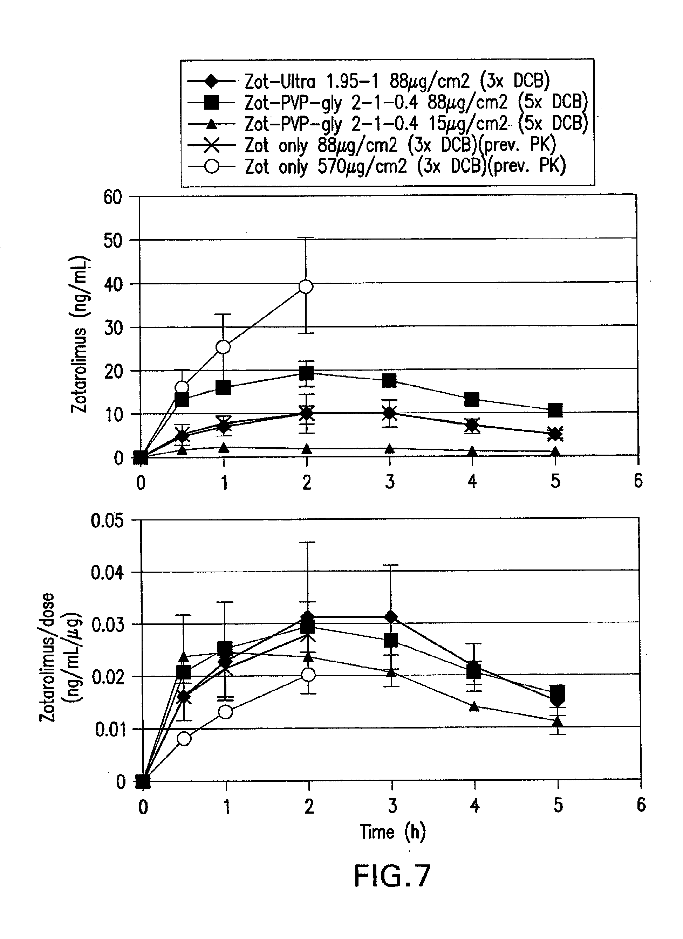

Blood zotarolimus concentrations increased as a function of time with Tmax ranging from 1-3 hours and Cmax from 2.1-39.4 ng/mL as a function of coating formulation as illustrated in Figure 7. It is interesting to note that blood concentrations appeared to exist more as a function of dose than excipient once the values were normalized. This trend indicated that excipients may serve more as both a binder and hydrophilic spacer than drug solubilizer. Blood data can be further modeled using a PK deconvolution / convolution model to predict blood concentration as function of total dose per each formulation as discussed above and shown in Figure 8.

**Claims**

1. A drug delivery balloon comprising:

   a balloon having a surface; and
   a coating disposed on at least a portion of the surface, wherein the coating includes a cytostatic therapeutic agent, an excipient, and a plasticizer, wherein at least 30% of the coating transfers from the balloon surface within two minutes after inflation of the balloon, and further wherein the coating produces a peak tissue concentration of the cytostatic therapeutic agent in the range of 10-1000 ng/mg of tissue approximately 24 hours after inflation of the balloon, and further wherein the cytostatic drug is everolimus, and the everolimus has a concentration on the balloon between 88 to 1500 ug/cm2 or between 100 to 600 ug/cm2, or wherein the cytostatic drug is zotarolimus, and the zotarolimus has a concentration on the balloon between 15 to 1500 ug/cm2.

2. The drug delivery balloon of claim 1, wherein at least 50%, at least 75% or at least 90% of the coating transfers from the balloon surface within two minutes after inflation of the balloon.

3. The drug delivery balloon of claim 1, wherein the excipient is polyvinylpyrrolidone, a polysorbate, or polyethylene glycol.

4. The drug delivery balloon of claim 1, wherein the plasticizer is selected from the group consisting of glycerol, ethanol, polyethylene glycol, propylene glycol, benzyl alcohol, N-methylpyrrolidone, Cremophor EL, dimethylsulfoxide, sorbitol, sucrose, water, or a blend thereof.

5. The drug delivery balloon of claim 1, wherein the balloon surface is modified, textured, roughened, or includes at least one channel, dimple, pore, or a combination thereof.

6. The drug delivery balloon of claim 1, wherein the coating transfers from the balloon surface to a tissue in a subject, and wherein the tissue is a blood vessel, a peripheral artery, or a blood vessel in an organ or a muscle.

7. The drug delivery balloon of claim 1, wherein the cytostatic drug is detectable in a tissue of a subject at least one week after delivery to a lumen in the subject.

8. The drug delivery balloon of claim 1 wherein the tissue concentration of everolimus decreases by more than 50% or more than 90% after about 72 hours post inflation of the balloon, or alternatively wherein the tissue concentration of zotarolimus decreases by more than 50% or more than 90% after about 72 hours post inflation of the balloon.

9. The drug delivery balloon of claim 1, wherein the everolimus released from the balloon has a concentration in the blood that does not exceed 179 ng/ml 24 hours post balloon inflation, or alternatively wherein the zotarolimus released from the balloon has a concentration in blood normalized to a total dosage that does not exceed 232 ng/ml 5 hours post balloon inflation or does not exceed a maximum concentration of 111 ng/ml 2 hours post balloon inflation.

10. The drug delivery balloon of claim 1, further comprising a stent disposed on the balloon, and/or wherein the balloon is a perfusion balloon.

11. The drug delivery balloon of claim 1, wherein the cytostatic drug is zotarolimus and the coating provides a tissue concentration half life in the range of 24 to 96 hours, or alternatively wherein the cytostatic drug is everolimus and the coating provides a tissue concentration half life in the range of 18 to 48 hours.

12. A drug delivery balloon comprising:

   a perfusion balloon having a surface; and
   a coating disposed on at least a portion of the surface, wherein the coating includes having an cytostatic therapeutic agent, an excipient, and a plasticizer, and further wherein at least 30% of the coating transfers from the balloon surface within ten minutes after inflation of the balloon, or within 5 minutes of inflation, or within 2 minutes of inflation or less, and further wherein the cytostatic drug is everolimus, and the everolimus has a concentration on the balloon between 88 to 1500 ug/cm2 or between 100 to 600 ug/cm2, or wherein the cytostatic drug is zotarolimus, and the zotarolimus has a concentration on the balloon between 15 to 1500 ug/cm2.

**13.** The drug delivery balloon of claim 12, wherein the cytostatic drug is zotarolimus, and optionally wherein the excipient is PVP and the plasticizer is glycerol.

**Patentansprüche**

**1.** Wirkstoffabgebender Ballon, umfassend:

einen Ballon mit einer Oberfläche; und
eine auf mindestens einem Teil der Oberfläche angeordnete Beschichtung, wobei die Beschichtung ein zytostatisches therapeutisches Mittel, einen Hilfsstoff und einen Weichmacher einschließt, wobei mindestens 30% der Beschichtung innerhalb von zwei Minuten nach Aufblähen des Ballons von der Ballonoberfläche transferieren, und des Weiteren wobei die Beschichtung eine Höchstkonzentration des zytostatischen therapeutischen Mittels im Gewebe im Bereich zwischen 10-1000 ng/mg des Gewebes ungefähr 24 Stunden nach Aufblähen des Ballons erzeugt, und des Weiteren wobei der zytostatische Wirkstoff Everolimus ist, und das Everolimus eine Konzentration auf dem Ballon zwischen 88 bis 1500 $\mu$g/cm$^2$ oder zwischen 100 bis 600 $\mu$g/cm$^2$ aufweist, oder wobei der zytostatische Wirkstoff Zotarolimus ist, und das Zotarolimus eine Konzentration auf dem Ballon zwischen 15 bis 1500 $\mu$g/cm$^2$ aufweist.

**2.** Wirkstoffabgebender Ballon nach Anspruch 1, wobei mindestens 50%, mindestens 75% oder mindestens 90% der Beschichtung innerhalb von zwei Minuten nach Aufblähen des Ballons von der Ballonoberfläche transferiert.

**3.** Wirkstoffabgebender Ballon nach Anspruch 1, wobei der Hilfsstoff Polyvinylpyrrolidon, ein Polysorbat, oder Polyethylenglycol ist.

**4.** Wirkstoffabgebender Ballon nach Anspruch 1, wobei der Weichmacher ausgewählt ist aus der Gruppe bestehend aus Glycerol, Ethanol, Polyethylenglycol, Propylenglycol, Benzylalkohol, N-Methylpyrrolidon, Cremophor EL, Dimethylsulfoxid, Sorbitol, Saccharose, Wasser, oder einer Mischung davon.

**5.** Wirkstoffabgebender Ballon nach Anspruch 1, wobei die Ballonoberfläche modifiziert, texturiert, geraut ist, oder mindestens einen Kanal, eine Vertiefung, eine Pore, oder eine Kombination davon einschließt.

**6.** Wirkstoffabgebender Ballon nach Anspruch 1, wobei die Beschichtung von der Ballonoberfläche zu einem Gewebe in einem Subjekt transferiert, und wobei das Gewebe ein Blutgefäß, eine periphere Arterie, oder ein Blutgefäß in einem Organ oder einem Muskel ist.

**7.** Wirkstoffabgebender Ballon nach Anspruch 1, wobei der zytostatische Wirkstoff in einem Gewebe von einem Subjekt mindestens eine Woche nach Zufuhr zu einem Lumen in dem Subjekt detektierbar ist.

**8.** Wirkstoffabgebender Ballon nach Anspruch 1, wobei die Gewebekonzentration von Everolimus um mehr als 50% oder mehr als 90% nach etwa 72 Stunden nach Aufblähen des Ballons abnimmt, oder alternativ, wobei die Gewebekonzentration von Zotarolimus um mehr als 50% oder mehr als 90% nach etwa 72 Stunden nach Aufblähen des Ballons abnimmt.

**9.** Wirkstoffabgebender Ballon nach Anspruch 1, wobei das von dem Ballon freigesetzte Everolimus eine Konzentration im Blut aufweist, die nicht 179 ng/ml 24 Stunden nach Ballonaufblähung übersteigt, oder alternativ, wobei das von dem Ballon freigesetzte Zotarolimus eine Konzentration im Blut aufweist, normalisiert auf eine Gesamtdosis, die nicht 232 ng/ml 5 Stunden nach Ballonaufblähung übersteigt oder nicht eine maximale Konzentration von 111 ng/ml 2 Stunden nach Ballonaufblähung übersteigt.

**10.** Wirkstoffabgebender Ballon nach Anspruch 1, des Weiteren umfassend einen auf dem Ballon angeordneten Stent, und/oder wobei der Ballon ein Perfusionsballon ist.

**11.** Wirkstoffabgebender Ballon nach Anspruch 1, wobei der zytostatische Wirkstoff Zotarolimus ist und die Beschichtung eine Halbwertszeit der Gewebekonzentration im Bereich von 24 bis 96 Stunden bereitstellt, oder alternativ, wobei der zytostatische Wirkstoff Everolimus ist und die Beschichtung eine Halbwertszeit der Gewebekonzentration im Bereich von 18 bis 48 Stunden bereitstellt.

**12.** Wirkstoffabgebender Ballon, umfassend:

einen Perfusionsballon mit einer Oberfläche; und
eine auf mindestens einen Teil der Oberfläche angeordnete Beschichtung, wobei die Beschichtung einschließt, dass sie ein zytostatisches therapeutisches Mittel, einen Hilfsstoff und einen Weichmachers aufweist, und des Weiteren, wobei mindestens 30% der Beschichtung von der Ballonoberfläche innerhalb von zehn Minuten nach Aufblähen des Ballons, oder innerhalb von fünf Minuten nach Aufblähen, oder innerhalb von zwei Minuten nach Aufblähen oder weniger transferieren, und des Weiteren, wobei der zytostatische Wirkstoff Everolimus ist und das Everolimus eine Konzentration auf dem Ballon zwischen 88 bis 1500 $\mu$g/cm$^2$ oder zwischen 100 bis 600 $\mu$g/cm$^2$ aufweist, oder wobei der zytostatische Wirkstoff Zotarolimus ist, und das Zotarolimus eine Konzentration auf dem Ballon zwischen 15 bis 1500 $\mu$g/cm$^2$ aufweist.

**13.** Wirkstoffabgebender Ballon nach Anspruch 12, wobei der zytostatische Wirkstoff Zotarolimus ist, und wobei gegebenenfalls der Hilfsstoff PVP ist und der Weichmacher Glycerol ist.

**Revendications**

**1.** Ballonnet pour délivrance de médicament, qui comprend :

- un ballonnet présentant une surface,
- et un revêtement placé sur au moins une partie de cette surface, lequel revêtement comporte un agent thérapeutique cytostatique, un excipient et un plastifiant,
et dans lequel
- une fraction d'au moins 30 % du revêtement se déplace de la surface du ballonnet en l'espace de deux minutes après le gonflement du ballonnet,
- et en outre, le revêtement donne un pic de concentration tissulaire de l'agent thérapeutique cytostatique, dans l'intervalle allant de 10 à 1000 nanogrammes par milligramme de tissu, à peu près 24 heures après le gonflement du ballonnet,
- et en outre, le médicament cytostatique est de l'évérolimus, présent sur le ballonnet en une concentration de 88 à 1500 $\mu$g/cm$^2$ ou de 100 à 600 $\mu$g/cm$^2$, ou le médicament cytostatique est du zotarolimus, présent sur le ballonnet en une concentration de 15 à 1500 $\mu$g/cm$^2$.

**2.** Ballonnet pour délivrance de médicament, conforme à la revendication 1, dans lequel une fraction d'au moins 50 %, d'au moins 75 % ou d'au moins 90 % du revêtement se déplace de la surface du ballonnet en l'espace de deux minutes après le gonflement du ballonnet.

**3.** Ballonnet pour délivrance de médicament, conforme à la revendication 1, dans lequel l'excipient est une poly (vinyl-pyrrolidone), un polysorbate ou un polyéthylèneglycol.

**4.** Ballonnet pour délivrance de médicament, conforme à la revendication 1, dans lequel le plastifiant est choisi dans l'ensemble formé par les glycérol, éthanol, polyéthylèneglycol, propylèneglycol, alcool benzylique, N-méthyl-pyrrolidone, Cremophor EL, diméthylsul-foxyde, sorbitol, saccharose et eau, et leurs mélanges.

**5.** Ballonnet pour délivrance de médicament, conforme à la revendication 1, dans lequel la surface du ballonnet est modifiée, est dotée d'une texture particulière ou d'une certaine rugosité, ou comporte au moins un canal, une bosse, un pore, ou une combinaison de telles structures.

**6.** Ballonnet pour délivrance de médicament, conforme à la revendication 1, dans lequel le revêtement se déplace de la surface du ballonnet vers un tissu chez un sujet, lequel tissu est un vaisseau sanguin, une artère périphérique, ou un vaisseau sanguin situé dans un organe ou dans un muscle.

**7.** Ballonnet pour délivrance de médicament, conforme à la revendication 1, avec lequel le médicament cytostatique est détectable dans un tissu chez un sujet au moins une semaine après sa délivrance dans la lumière d'un conduit chez le sujet.

**8.** Ballonnet pour délivrance de médicament, conforme à la revendication 1, avec lequel la concentration tissulaire d'évérolimus est abaissée de plus de 50 %, ou de plus de 90 %, au bout d'environ 72 heures après le gonflement du ballonnet, ou bien avec lequel la concentration tissulaire de zotarolimus est abaissée de plus de 50 %, ou de plus de 90 %, au bout d'environ 72 heures après le gonflement du ballonnet.

9. Ballonnet pour délivrance de médicament, conforme à la revendication 1, avec lequel l'évérolimus relâché par le ballonnet se trouve dans le sang en une concentration qui ne dépasse pas 179 ng/mL 24 heures après le gonflement du ballonnet, ou bien avec lequel le zotarolimus relâché par le ballonnet se trouve dans le sang en une concentration, normalisée à la dose totale, qui ne dépasse pas 232 ng/mL 5 heures après le gonflement du ballonnet, ou ne dépasse pas une concentration maximale de 111 ng/mL 2 heures après le gonflement du ballonnet.

10. Ballonnet pour délivrance de médicament, conforme à la revendication 1, qui comprend en outre un stent disposé sur le ballonnet et/ou lequel ballonnet est un ballonnet à perfusion.

11. Ballonnet pour délivrance de médicament, conforme à la revendication 1, dans lequel le médicament cytostatique est du zotarolimus et le revêtement assure, pour la concentration tissulaire, une demi-vie située dans l'intervalle allant de 24 à 96 heures, ou bien dans lequel le médicament cytostatique est de l'évérolimus et le revêtement assure, pour la concentration tissulaire, une demi-vie située dans l'intervalle allant de 18 à 48 heures.

12. Ballonnet pour délivrance de médicament, qui comprend :

   - un ballonnet à perfusion présentant une surface,
   - et un revêtement placé sur au moins une partie de cette surface, lequel revêtement comporte un agent thérapeutique cytostatique, un excipient et un plastifiant,
   et dans lequel
   - une fraction d'au moins 30 % du revêtement se déplace de la surface du ballonnet en l'espace de dix minutes après le gonflement du ballonnet, ou en l'espace de cinq minutes après ce gonflement, ou en l'espace de deux minutes ou moins après ce gonflement,
   - et en outre, le médicament cytostatique est de l'évérolimus, présent sur le ballonnet en une concentration de 88 à 1500 $\mu$g/cm$^2$ ou de 100 à 600 $\mu$g/cm$^2$, ou le médicament cytostatique est du zotarolimus, présent sur le ballonnet en une concentration de 15 à 1500 $\mu$g/cm$^2$.

13. Ballonnet pour délivrance de médicament, conforme à la revendication 12, dans lequel le médicament cytostatique est du zotarolimus, et dans lequel, en option, l'excipient est une poly(vinyl-pyrrolidone) et le plastifiant est du glycérol.

FIG.1A

FIG.1B

FIG.2A

FIG.2B

**FIG.3A**

**FIG.3B**

Normalized tissue uptake ng/mg/μg

FIG.4

FIG.5

FIG.6

**Legend:**
- Zot-Ultra 1.95-1 88µg/cm² + BMS (CA)
- Zot-PVP-Gly 2-1-0.4 88µg/cm² + BMS (CA)
- Zot-PVP-Gly 2-1-0.4 88µg/cm² (CA, MA)
- Zot-PVP-Gly 2-1-0.4 15µg/cm² + BMS (CA)
- Zot-PVP-Gly 2-1-0.4 15µg/cm² (CA, MA)
- Zot only 88µg/cm² + BMS (previous, CA)

*Greater than all arms except Zot-PVP 88µg/cm² + BMS

#Greater than Zot-PVP 88µg/cm², Zot-PVP 15µg/cm² + BMS, Zot-PVP 15µg/cm²

ANOVA, $p < 0.05$

Note: MA = mammary arteries, no difference observed in uptake between CA and MA

Top chart (30min): Zotarolimus (ng/mg); values: 498*, 414#, 50, 16, 11, 153

Bottom chart (7d): Zotarolimus (ng/mg); 7d target > 0.029 ng/mg; values: 1.8, 0.4, 0.1, 0.03, 0.1, 0.2

FIG.7

FIG.8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009051614 A
- US 6669980 B, Hansen
- US 7241344 B, Worsham
- US 20040234748 A, Stenzel
- US 5951514 A, Sahota
- US 5370617 A, Sahota
- US 5542925 A, Orth
- US 5989218 A, Wasicek
- US 6406457 B, Wang
- US 6500148 B, Pinchuk
- US 6991617 B, Hektner
- US 7273417 B, Wang
- US 51023402 B, Dror
- US 6129705 A, Grantz
- US 5370614 A, Amundson